(19) **Europäisches Patentamt / European Patent Office / Office européen des brevets**

(11) **EP 1 332 675 B2**

(12) # NEW EUROPEAN PATENT SPECIFICATION
### After opposition procedure

(45) Date of publication and mention of the opposition decision:
**07.11.2012 Bulletin 2012/45**

(45) Mention of the grant of the patent:
**27.04.2005 Bulletin 2005/17**

(21) Application number: **03250329.4**

(22) Date of filing: **18.01.2003**

(51) Int Cl.:
*A01N 43/80* (2006.01)  *A01N 43/80* (2006.01)
*A01N 43/50* (2006.01)  *A01N 43/40* (2006.01)
*A01N 39/00* (2006.01)  *A01N 37/36* (2006.01)
*A01N 37/34* (2006.01)  *A01N 37/10* (2006.01)
*A01N 37/06* (2006.01)  *A01N 31/04* (2006.01)

(54) **Synergistic microbicidal combination**

Synergistische mikrobizide Kombination

Association microbicide synergique

(84) Designated Contracting States:
**DE FR GB IT**

(30) Priority: **31.01.2002 US 352621 P**

(43) Date of publication of application:
**06.08.2003 Bulletin 2003/32**

(60) Divisional application:
**04078581.8 / 1 525 797**
**04255357.8 / 1 502 508**
**04255358.6 / 1 488 699**
**04257939.1 / 1 523 887**
**04257944.1 / 1 527 684**
**04257945.8 / 1 527 685**
**04257946.6 / 1 621 076**

(73) Proprietor: **ROHM AND HAAS COMPANY**
**Philadelphia, PA 19106-2399 (US)**

(72) Inventors:
• **Warwick Eileen Fleck**
**Lansdale,**
**Pennsylvania 19446 (US)**
• **Diehl, Megan Anne**
**Line Lexington,**
**Pennsylvania 18932 (US)**

(74) Representative: **Kent, Venetia Katherine et al**
**Patent Outsourcing Limited**
**1 King Street**
**Bakewell**
**Derbyshire DE45 1DZ (GB)**

(56) References cited:
EP-A- 0 398 795   EP-A- 0 435 439
EP-A- 0 450 916   EP-A- 0 508 457
EP-A- 0 544 418   EP-A- 0 745 324
EP-A- 0 773 281   EP-A- 0 923 867
EP-A- 1 044 609   EP-A- 1 082 901
EP-A- 1 084 619   WO-A-01/00022
WO-A-01/19325   WO-A-02/17716
WO-A-99/08530   WO-A1-00/10393
GB-A- 2 138 799   US-A- 5 227 156
US-A- 6 114 366   US-A- 6 121 302

• **DATABASE WPI Section Ch, Week 199245 Derwent Publications Ltd., London, GB; Class C02, AN 1992-369386 XP002249377 & JP 04 270203 A (SHINTO PAINT CO LTD) 25 September 1992 (1992-09-25)**

Remarks:
The file contains technical information submitted after the application was filed and not included in this specification

EP 1 332 675 B2

**Description**

**BACKGROUND**

This invention relates to synergistic microbicidal combinations and, in particular, to synergistic microbicidal combinations of 2-methyl-3-isothiazolone with the commercial microbicide benzoic acid, where the resulting composition is substantially free of halogenated 3-isothiazolone.

Kathon™ CG biocide (3/1 ratio of 5-chloro-2-methyl-3-isothiazolone to 2-methyl-3-isothiazolone) and Neolone™ 950 bactericide (2-methyl-3-isothiazolone) are highly effective preservatives and may be used in combination with a variety of commercially available preservatives to prevent microbial contamination in personal care applications, such as cosmetics and toiletries (see "Kathon™ CG Cosmetic and Toiletry Preservative" Bulletin CS-663, September 1997, and " Neolone™ 950 Preservative for Personal Care" Bulletin CS-707, May 2001, Rohm and Haas Company, Philadelphia, PA). U.S. Patent No. 5,591,759 discloses the use of 1,2-dibromo-2,4-dicyanobutane to stabilize isothiazolone mixtures of 5-chloro-2-methyl-3-isothiazolone/2-methyl-3-isothiazolone (9/1 ratio). Patent application WO 01/50855 discloses preservative formulations for extending the lifetime of cut flowers based on preservative formulations containing acrylic copolymer additives, saccharide derivatives and antimicrobial mixtures, including those based on 2-methyl-3-isothiazolone in the presence of citric acid/citrates.

Many other microbicidal agents are known. These are commercially available for the control of microorganisms in a variety of loci. Sometimes, many microbicides cannot provide effective antimicrobial control even at high use concentrations due to weak activity against certain microorganisms. Without effective microorganism control, loss of product, inferior product, production time loss, health hazard and other problems may occur in the locus treated. There is a need for a method to control various microorganisms that does not rely on high use levels of combinations of different microbicidal agents, but still provides effective overall control of the microorganisms that is both quick and long lasting.

Mixtures of isothiazolones and benzoic acid are known from EP-A-0 923 867 and EP-A-1 044 609, but such mixtures are disclosed to comprise halogenated 3-isothiazolone.

The problem addressed by the present invention is to overcome deficiencies of previous microbicide combinations by providing a combination of microbicidal agents that is more effective than the individual microbicides used alone and that can be used at lower overall levels while providing efficacy similar to the original individual microbicide levels.

**STATEMENT OF INVENTION**

The present invention provides a microbicidal composition comprising a synergistic mixture, the first component of which is 2-methyl-3-isothiazolone, and the second component of which is the commercial microbicide benzoic acid; wherein the ratio of the first component to the second component is from 1/0.13 to 1/67; and wherein the composition is substantially free of halogenated 3-isothiazolone.

**DETAILED DESCRIPTION**

We have discovered that 2-methyl-3-isothiazolone (**MI**) may be combined with benzoic acid to provide enhanced microbicidal efficacy at a combined active ingredient level lower than that of the combined individual 3-isothiazolone or commercial microbicide effective use levels, where the resulting composition is substantially free of halogenated 3-isothiazolone. Preferably the microbicidal compositions are also substantially free of metal salt stabilizers, such as nitrate or magnesium salts; these "salt-free" microbicidal compositions are especially useful to protect personal care compositions against microbial contamination.

It has been discovered that mixtures of **MI** with the commercial microbicide of the present invention in a ratio of 1/0.13 to 1/67 result in synergistic microbicidal activities against a wide range of microorganisms. Synergy occurs when the disruptive interaction on the organisms treated by the two compounds together is greater than the sum of such interactions of both compounds when used alone. Such synergy does not arise from the expected activity of the components when added together.

As used herein, the following terms have the designated definitions, unless the context clearly indicates otherwise. The term "microbicide" refers to a compound capable of inhibiting the growth of or controlling the growth of microorganisms at a locus; microbicides include bactericides, fungicides and algaecides. The term "microorganism" includes, for example, fungi (such as yeast and mold), bacteria and algae. The term "locus" refers to an industrial system or product subject to contamination by microorganisms. The following abbreviations are used throughout the specification: ppm = parts per million by weight (weight/weight), mL = milliliter, ATCC = American Type Culture Collection, and MIC = minimum inhibitory concentration. Unless otherwise specified, ranges listed are to be read as inclusive and combinable, temperatures are in degrees centigrade (°C), and references to percentages (%) are by weight. "Salt-free" means that the composition contains zero or up to 0.5%, preferably zero or up to 0.1%, and more preferably zero or up to 0.01%, of metal salt, based on weight of the composition.

The microbicidal compositions of the present invention are substantially free of halogenated 3-isothiazolone; that is, zero or up to 3%, preferably zero or up to 1% and more preferably zero or up to 0.5%, of halogenated 3-isothiazolone may be present, based on combined weight of halogenated 3-isothiazolone and 2-methyl-3-isothiazolone. Microbicidal compositions dependent on the presence of halogenated 3-isothiazolone are susceptible to chemical degradation and may require additional stabilizer components, such as the aforementioned metal salt stabilizers; salt stabilizers sometimes create unacceptable properties in finished formulations. For this reason it is desirable to provide microbicide formulations substantially free of halogenated 3-isothiazolone, but that still provide the degree of antimicrobial protection provided by the halogenated 3-isothiazolones; such are the microbicidal compositions of the present invention that are based on 2-methyl-3-isothiazolone, which do not require metal stabilizers.

**MI** may be used in the synergistic mixtures of the present invention "as is" or may first be formulated with a solvent or a solid carrier. Suitable solvents include, for example, water; glycols, such as ethylene glycol, propylene glycol, diethylene glycol, dipropylene glycol, polyethylene glycol, and polypropylene glycol; glycol ethers; alcohols, such as methanol, ethanol, propanol, phenethyl alcohol and phenoxypropanol; ketones, such as acetone and methyl ethyl ketone; esters, such as ethyl acetate, butyl acetate, triacetyl citrate, and glycerol triacetate; carbonates, such as propylene carbonate and dimethyl carbonate; and mixtures thereof. It is preferred that the solvent is selected from water, glycols, glycol ethers, esters and mixtures thereof. Suitable solid carriers include, for example, cyclodextrin, silicas, diatomaceous earth, waxes, cellulosic materials and charcoal. It is preferred that **MI** is formulated in water.

The second microbicide component of the combinations of the present invention is the well-known and generally commercially available microbicide benzoic acid. The ratio of **MI** to the second microbicide component is 1/0.13 to 1/67. The microbicide may be used in the synergistic mixtures of the present invention "as is" or may first be formulated together with a suitable solvent, a solid carrier or as a dispersion. Suitable solvents and solid carriers are those described above for **MI**.

When a water-insoluble second microbicide component is formulated in a solvent, the formulation may optionally contain surfactants. When such formulations contain surfactants, they are generally in the form of emulsive concentrates, emulsions, microemulsive concentrates, or microemulsions. Emulsive concentrates form emulsions upon the addition of a sufficient amount of water. Microemulsive concentrates form microemulsions upon the addition of a sufficient amount of water. Such emulsive and microemulsive concentrates are generally well known in the art; it is preferred that such formulations are free of surfactants. U.S. Patent No. 5,444,078 may be consulted for further general and specific details on the preparation of various microemulsions and microemulsive concentrates.

Any formulation of **MI** may be used with any formulation of the second microbicide component in the synergistic mixtures of the present invention. When both the **MI** and the second microbicide component are each first formulated with a solvent, the solvent used for **MI** may be the same as or different from the solvent used to formulate the other commercial microbicide. It is preferred that the two solvents are miscible. In the alternative, the **MI** and the other microbicide may be combined directly and then a solvent added to the mixture.

Those skilled in the art will recognize that the **MI** and the second microbicide component of the present invention may be added to a locus sequentially, simultaneously, or may be combined before being added to the locus. It is preferred that the **MI** and the second microbicide component be added to a locus simultaneously or combined prior to being added to the locus. When the microbicides are combined prior to being added to a locus, such combination may optionally contain adjuvants, such as, for example, solvent, thickeners, anti-freeze agents, colorants, sequestrants (such as ethylenediaminetetraacetic acid, ethylenediaminedisuccinic acid, iminodisuccinic acid and salts thereof), dispersants, surfactants, stabilizers, scale inhibitors and anti-corrosion additives.

The microbicidal compositions of the present invention can be used to inhibit the growth of microorganisms by introducing a microbicidally effective amount of the compositions onto, into, or at a locus subject to microbial attack. Suitable loci include, for example: cooling towers; air washers; boilers; mineral slurries; wastewater treatment; ornamental fountains; reverse osmosis filtration; ultrafiltration; ballast water; evaporative condensers; heat exchangers; pulp and paper processing fluids; plastics; emulsions; dispersions; paints; latexes; coatings, such as varnishes; construction products, such as mastics, caulks, and sealants; construction adhesives, such as ceramic adhesives, carpet backing adhesives, and laminating adhesives; industrial or consumer adhesives; photographic chemicals; printing fluids; household products, such as bathroom and kitchen cleaners; cosmetics; toiletries; shampoos; soaps; detergents; industrial cleaners; floor polishes; laundry rinse water; metalworking fluids; conveyor lubricants; hydraulic fluids; leather and leather products; textiles; textile products; wood and wood products, such as plywood, chipboard, flakeboard, laminated beams, oriented strandboard, hardboard, and particleboard; petroleum processing fluids; fuel; oilfield fluids, such as injection water, fracture fluids, and drilling muds; agriculture adjuvant preservation; surfactant preservation; medical devices; diagnostic reagent preservation; food preservation, such as plastic or paper food wrap; pools; and spas.

Preferably, the microbicidal compositions of the present invention are used to inhibit the growth of microorganisms at a locus selected from one or more of emulsions, dispersions, paints, latexes, household products, cosmetics, toiletries, shampoos, soaps, detergents and industrial cleaners. In particular, the microbicidal compositions are useful in personal care applications, such as hair care (for example, shampoo and dyes) and skin care (for example, sunscreens, cosmetics, soaps, lotions and creams) formulations.

When the synergistic compositions of the present invention are used in personal care compositions, the formulated compositions may also comprise one or more ingredients selected from UV radiation-absorbing agents, surfactants, rheology modifiers or thickeners, fragrances, moisturizers, humectants, emollients, conditioning agents, emulsifiers, antistatic aids, pigments, dyes, tints, colorants, antioxidants, reducing agents and oxidizing agents.

Suitable UV radiation-absorbing agents (including chemical absorbers and physical blockers) include, for example, oxybenzone, dioxybenzone, sulisobenzone, menthyl anthranilate, para-aminobenzoic acid, amyl para-dimethylaminobenzoic acid, octyl para-dimethylaminobenzoate, ethyl 4-bis(hydroxypropyl)aminobenzoate, polyethylene glycol (PEG-25) para-aminobenzoate, diethanolamine para-methyoxycinnamate, 2-ethoxyethyl para-methoxycinnamate, ethylhexyl para-methoxycinnamate, octyl para-methoxycinnamate, isoamyl para-methoxycinnamate, 2-ethylhexyl-2-cyano-3,3-diphenylacrylate, 2-ethylhexyl salicylate, homomenthyl salicylate, glyceryl aminobenzoate, triethanolamine salicylate, digalloyl trioleate, lawsone with dihydroxyacetone, 2-phenylbenzimidazole-5-sulfonic acid, 4-methylbenzylidine camphor, avobenzone, titanium dioxide and zinc oxide. Alternatively, UV radiation-absorbing agents such as triazines, benzotriazoles, vinyl group-containing amides, cinnamic acid amides and sulfonated benzimidazoles may also be used.

Suitable surfactants include, for example, nonionic, anionic, cationic and amphoteric surfactants and mixtures thereof; such as PPG 28 Buteth 35, PEG 75 lanolin, perfluoropolymethyl isopropyl ether, octoxynol-9, PEG-25 hydrogenated castor oil, polyethylene terephthalate, polyethylene glycol 25 glyceryl trioleate, oleth-3 phosphate, PPG-5-ceteth-10 phosphate, PEG-20 methyl glucose ether, glycereth-7-triacetate and N-alkyl substituted lactam (such as N-octyl pyrrolidone).

Suitable thickeners or rheology modifiers include, for example, hydrophobically modified nonionic ethoxylated urethanes, polycarboxylic acid thickeners such as acrylates/steareth-20 methacrylate copolymer, carbomers, acrylates copolymer and acrylates $C_{10-30}$ alkyl acrylate crosspolymer.

The personal care compositions improved by the method of this invention include, for example:

(a) hair care formulations, including shampoos, hair dyes, hair conditioners, gels, mousses and hair sprays; and
(b) skin care and nail care formulations, including nail coatings, cosmetics, astringents, depilatories, facial make-up formulations, sunscreens and sunblocks, premoistened wipes, hand creams, hand and body soaps, and hand and body lotions.

The cosmetic formulations typically contain water, film forming materials, emulsifiers, softeners, emollients, oils, stabilizers, thickeners, neutralizers, perfume, colorants, pigments and combinations thereof. The sunscreen formulations typically contain UV radiation-absorbing agents, water, film forming materials, emulsifiers, emollients, waterproofing agents, oils, stabilizers, thickeners, preservatives, perfume, colorants, insecticides, humectants and combinations thereof.

Optionally, other additives, such as additional film forming agents, plasticizers, antifoaming agents, leveling aids, excipients, vitamins, natural extracts, proteins, sequestrants, dispersants, antioxidants, suspending agents and solvents may be added to the personal care formulations described above. Suitable solvents include, for example, $C_1$-$C_{12}$ straight or branched chain alcohols such as ethanol, isopropanol, or propanol; alkyl esters such as ethyl acetate; ketones; and combinations thereof.

The specific amount of the synergistic combinations necessary to inhibit or control the growth of microorganisms in a locus depends upon the particular compounds in the combination and particular locus to be protected. Typically, the amount of the synergistic combinations of the present invention to control the growth of microorganisms in a locus is sufficient if it provides from 0.1 to 10,000 ppm active ingredient of the synergistic mixture in the locus. It is preferred that the synergistic mixture be present in an amount of 0.5 to 5000 ppm, and more preferably from 1 to 3000 ppm, in the locus.

Microorganisms evaluated for effectiveness of control by the compositions of the present invention include bacterial (*P. aeruginosa* and *S. aureus)* and fungal (*A. niger* and *C. albicans*) microorganisms, in particular Gram (-) bacteria and Gram (+) bacteria, as well as yeast and mold.

Combinations of **MI** with benzoic acid were particulary effective against Gram (-) bacteria. Combinations of **MI** with benzoic acid, were also particulary effective against yeast. Combinations of **MI** with benzoic acid, were effective against both bacterial and fungal microorganisms.

When benzoic acid is used in the synergistic mixtures of the present invention, the ratio of **MI** to benzoic acid is from 1/0.13 to 1/67.

Some embodiments of the invention are described in detail in the following Examples. All ratios, parts and percentages are expressed by weight unless otherwise specified, and all reagents used are of good commercial quality unless otherwise specified. Abbreviations used in the Examples and Tables are listed below with the corresponding descriptions:

**MI** = 2-methyl-3-isothiazolone
**SI** = synergy index
**MIC** = minimum inhibitory concentration

## EXAMPLES

The synergism of the combinations of the present invention was demonstrated by testing a wide range of concentrations and ratios of the compounds.

Synergism was determined by an industrially accepted method described by Kull, F.C.; Eisman, P.C.; Sylwestrowicz, H.D. and Mayer, R.L., in Applied Microbiology 9:538-541 (1961), using the ratio determined by the formula:

$$Q_a/Q_A + Q_b/Q_B = \text{Synergy Index ("SI")}$$

wherein:

$Q_A$ = concentration of compound A (first component) in ppm, acting alone, which produced an end point (MIC of Compound A).

$Q_a$ = concentration of compound A in ppm, in the mixture, which produced an end point.

$Q_B$ = concentration of compound B (second component) in ppm, acting alone, which produced an end point (MIC of Compound B).

$Q_b$ = concentration of compound B in ppm, in the mixture, which produced an end point.

When the sum of $Q_a/Q_A$ and $Q_b/Q_B$ is greater than one, antagonism is indicated. When the sum is equal to one, additivity is indicated, and when less than one, synergism is demonstrated. The lower the **SI**, the greater the synergy shown by that particular mixture. The minimum inhibitory concentration (MIC) of a microbicide is the lowest concentration tested under a specific set of conditions that prevents the growth of added microorganisms.

Synergy tests were conducted using standard microtiter plate assays with media designed for optimal growth of the test microorganism. Minimal salt medium supplemented with 0.2% glucose and 0.1% yeast extract (M9GY medium) was used for testing bacteria; Potato Dextrose Broth (PDB medium) was used for testing yeast and mold. In this method, a wide range of combinations of microbicides was tested by conducting high resolution MIC assays in the presence of various concentrations of **MI.** High resolution MICs were determined by adding varying amounts of microbicide to one column of a microtitre plate and doing subsequent ten-fold dilutions using an automated liquid handling system to obtain a series of endpoints ranging from 2 ppm to 10,000 ppm active ingredient. The synergy of the combinations of the present invention was determined against two bacteria, *Staphylococcus aureus* (*S. aureus* -- ATCC #6538) or *Pseudomonas aeruginosa* (*P. aeruginosa* -- ATCC #9027), a yeast, *Candida albicans* (*C. albicans* -- ATCC 10231), and a mold, *Aspergillus niger* (*A. niger* -- ATCC 16404). The bacteria were used at a concentration of about $5 \times 10^6$ bacteria per mL and the yeast and mold at $5 \times 10^5$ fungi per mL. These microorganisms are representative of natural contaminants in many consumer and industrial applications. The plates were visually evaluated for microbial growth (turbidity) to determine the MIC after various incubation times at 25°C (yeast and mold) or 30°C (bacteria).

The test results for demonstration of synergy of the microbicide combinations of the present invention are shown below in Tables 1 through 9. In each test, First Component (A) was **MI** and the Second Component (B) was the other commercial microbicide. Each table shows the specific combinations of **MI** and the second component; results against the microorganisms tested with incubation times; the end-point activity in ppm measured by the MIC for **MI** alone ($Q_A$), for the second component alone ($Q_B$), for **MI** in the mixture ($Q_a$) and for second component in the mixture ($Q_b$); the calculated **SI** value; and the range of synergistic ratios for each combination tested (**MI**/second component or **A/B**).

Table 1

| First Component (A) = 2-methyl-3-isothiazolone<br>Second Component (B) = benzoic acid |
| --- |

(continued)

| Microorganism | $Q_a$ | $Q_b$ | SI | A/B |
|---|---|---|---|---|
| *A. niger*<br>(1 week) | 0 | 10000 | 1.00 | ----- |
| | 50 | 10000 | 1.17 | 1/200 |
| | 200 | 10000 | 1.67 | 1/50 |
| | 300 | 0 | 1.00 | ----- |
| *C. albicans*<br>(48 hours) | 0 | 5000 | 1.00 | ----- |
| | 125 | 2000 | 1.03 | 1/16 |
| | 125 | 1000 | 0.83 | 1/8 |
| | 125 | 800 | 0.79 | 1/6.4 |
| | 125 | 600 | 0.75 | 1/4.8 |
| | 125 | 500 | 0.73 | 1/4 |
| | 125 | 400 | 0.71 | 1/3.2 |
| | 125 | 300 | 0.69 | 1/2.4 |
| | 125 | 200 | 0.67 | 1/1.6 |
| | 125 | 100 | 0.65 | 1/0.8 |
| | 125 | 80 | 0.64 | 1/0.64 |
| | 125 | 60 | 0.64 | 1/0.48 |
| | 125 | 50 | 0.64 | 1/0.4 |
| | 125 | 40 | 0.63 | 1/0.32 |
| | 150 | 30 | 0.76 | 1/0.2 |
| | 150 | 20 | 0.75 | 1/0.13 |
| | 200 | 0 | 1.00 | ----- |
| *S. aureus*<br>(72 hours) | 0 | 6000 | 1.00 | ----- |
| | 15 | 6000 | 1.25 | 1/400 |
| | 50 | 5000 | 1.67 | 1/100 |
| | 60 | 0 | 1.00 | ----- |
| *P. aeruginosa*<br>(72 hours) | 0 | 6000 | 1.00 | ----- |
| | 15 | 2000 | 1.08 | 1/133 |
| | 15 | 1000 | 0.92 | 1/67 |
| | 15 | 800 | 0.88 | 1/53 |
| | 15 | 600 | 0.85 | 1/40 |
| | 15 | 500 | 0.83 | 1/33 |
| | 15 | 400 | 0.82 | 1/27 |
| | 15 | 300 | 0.80 | 1/20 |
| | 20 | 0 | 1.00 | ----- |

The synergistic ratios of **MI**/benzoic acid range from 1/0.13 to 1/67. The **MI**/benzoic acid combination showed enhanced control of Gram (-) bacteria and yeast.

## Claims

1.   A microbicidal composition comprising a synergistic mixture, the first component of which is 2-methyl-3-isothiazolone, and the second component of which is the commercial microbiocide benzoic acid; wherein the ratio of the first component to the second component is from 1/0.13 to 1/67; and wherein the composition contains no more than 3% of halogenated 3-isothiazolone, based on combined weight of halogenated 3-isothiazolone and 2-methyl-3-isothiazolone.

**EP 1 332 675 B2**

**Patentansprüche**

1. Mikrobizide Zusammensetzung, die eine synergistische Mischung umfasst, deren erste Komponente 2-Methyl-3-isothiazolon ist und deren zweite Komponente die handelsübliche mikrobizide Benzoesäure ist; wobei das Verhältnis der ersten Komponente zur zweiten Komponente von 1/0,13 bis 1/67 reicht; und wobei die Zusammensetzung nicht mehr als 3 % halogeniertes 3-Isothiazolon enthält, bezogen auf das Gesamtgewicht von halogeniertem 3-Isothiazolon und 2-Methyl-3-isothiazolon.

**Revendications**

1. Composition microbicide comprenant un mélange synergique, dont le premier composant est la 2-méthyl-3-isothiazolone, et dont le second composant est le microbicide du commerce acide benzoïque ; dans laquelle le rapport du premier composant au second composant est de 1/0,13 à 1/67 ; et dans laquelle la composition ne contient pas plus de 3 % de 3-isothiazolone halogénée, rapporté à la masse combinée de 3-isothiazolone halogénée et de 2-méthyl-3-isothiazolone.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5591759 A
- WO 0150855 A
- EP 0923867 A
- EP 1044609 A
- US 5444078 A

### Non-patent literature cited in the description

- Kathon™ CG Cosmetic and Toiletry Preservative. Bulletin CS-663. September 1997
- Neolone™ 950 Preservative for Personal Care. Bulletin CS-707. Rohm and Haas Company, May 2001
- **KULL, F.C. ; EISMAN, P.C. ; SYLWESTROWICZ, H.D. ; MAYER, R.L.** *Applied Microbiology,* 1961, vol. 9, 538-541